# EUROPEAN PATENT APPLICATION

(11) **EP 1 669 452 A1**
(43) Date of publication of application: **14.06.2006**
(21) Application number: 04772380.4
(22) Date of filing: 23.08.2004
(51) Int. Cl.: C12N 15/12, C12N 5/10, C12N 1/15, C12N 1/19, C12N 1/21, A61K 35/76, A61K 48/00, A61P 29/00

(54) **PROMOTER OF SYNOVIOLIN GENE**

(30) Priority: 21.08.2003 JP 2003297913
(71) Applicant: Locomogene, Inc., Tokyo 105-0001 (JP)
(72) Inventor: NAKAJIMA, Toshihiro, Kohoku Garden Hills A-503, Yokohama-shi, Kanagawa 224-0001 (JP); AMANO, Tetsuya, Kawasaki-shi, Kanagawa 214-0005 (JP); TSUCHIMOCHI, Kaneyuki, Kawasaki-shi, Kanagawa 214-0035 (JP); YAMASAKI, Satoshi, Ishikawazaka Mansyon 305, Yokohama-shi, Kanagawa 225-0003 (JP); YAGISHITA, Naoko, Yokohama-shi, Kanagawa 235-0053 (JP)
(74) Representative: Woods, Geoffrey Corlett
(86) International application number: PCT/JP2004/012424
(87) International publication number: WO 2005/019456

(57) **Abstract**

The present invention provides a promoter of the synoviolin gene comprising at least the nucleotide sequence of nucleotides 2120-2130 in the nucleotide sequence represented by SEQ ID: NO 1 or 2, or comprising at least the nucleotide sequence of the region of nucleotides 1-2201, 969-2201, 1142-2201, 1699-2201, 1880-2201, 2002-2201, 2094-2201 or 2118-2201 in the nucleotide sequence represented by SEQ ID: NO 1 or 2;or a method for regulating transcription activity **characterized in that** the activity of the promoter is inhibited or enhanced.

SEQUENCE LISTING

<110>  Locomogene, Inc.

<120>  Synobiolin promoter

<130>  N97359

<140>  EP 04772380.4
<141>  2004-08-23

<150>  PCT/JP2004/012424
<151>  2004-08-23


<150>  2003-297913
<151>  2003-09-02

<160>  7

<170>  PatentIn version 3.2

<210>  1
<211>  3046
<212>  DNA
<213>  Mus musclus

<400>  1
gcaagagacc ttattttgtt tttcgagaca gggtttctct gtgtagccct ggctgtccta      60

gaactcactc tgtagaccag gctggcctcg aactcagaaa tccgcctgcc tctgcctccc     120

gagtgctggg attaaaggta ggcgccacca cgcccagctt tttttttttt agataggatc     180

tcactctata gctgtacgct ggcctcagat ttatgatgct cttcctgcct cagtctccca     240

attttctggg attgtaggag tgggccacta tgctctgctc actacatgat ttcagaggtt     300

gagtagacct gaactgaaga ccagacaagg gagccctccc tcgacatctt ggggccaggg     360

aagttgaagc cataggatca gaggaaatgt ggcaagaaaa aaggccaaca tggacacaga     420

acttaaataa aaacagacag aggaagtaag acagatatat acctggggga gaggagggat     480

tgccacaaaa tgtaggagat tttcaagaat gggggaggat gagtgtgtag ggttaaaggt     540

agccagtaga agttcatagc tagccttatg gaggaaggaa aggggagcca tctcgggatg     600

ttaactgtta aagacaacag gtggtggtga agatggctga gaccaagagc acagggctga     660

ggggcagaca ggcactgaca ctgctaccct ttaatacagt tcctcctgtt gtgatcccca     720

accataatta cttcgttgct acttcataac tgtaattttg ctagttatga attgtaagta     780

aacgtctgat atgcaggata tctcatttgt gacccctgtg taacggtttg attcccaaag     840

ggcttacgac tcacaggttg agagccagcc actgccttaa agtcgtctag aatcagtttt     900

ctttcttttt tgacagacaa gatgtttaat tccgttgtac tgaaggaaag ccattttatg     960

tatttttctt aagtgctcta tcagtaatga caattctgaa agcccctgtg ttatatttta    1020

acaacacagt cacctccggt tctgtattca ctgtccgtgt tgtgactccc acagtataaa 1080
ttcctccagt tgatcttcat gaattcttat atttgatccc cccccccctt aggcctctga 1140
attccgagtg agtccgagtt aaaaatggga ggagcaccct ctagctgata aacctgggta 1200
atgaggtgtc cgctttcagt ttccattctg tacgcgacta tactgcttgt gtgagcccta 1260
acagacagaa tcagctcaga acaaagggtc tggctatctc ccagggatga acacgcacgc 1320
cgactgagct tttggggtgt tgaaaagtca acgccttcgc acagaactct ccaccccaac 1380
ctagaaataa ctggcgttct gttttatgtc agtccggaca cgcaagcact gctccttttg 1440
cgggccccgt aagcatcccc ccaggcggga tagggatccc cggcctatgg actgcgcttt 1500
ctcagctggc atccagctgc cttggcaccc agtccggggc cactctgcct acagacccta 1560
gcaaccactc acctgctttt ctttccctat aggccagaaa tttttccttt cttttctcat 1620
tggtccgcgt aactttatcg caaccaatcg gcggtacacg ggaacaaact cactcctaca 1680
caacctgcgt tggggggagg taacctggga agacctatat ctgttttctg caccgctatt 1740
tttttccgag aagcacttaa cttcttaccg tgtcgtagct atccctggaa tgaggcgctt 1800
acacatttta tttctttcat gcctgacata aagtctggcc cttgctcgct cctgcccccc 1860
gtccaaatgg ctcggcccgc ggaacgccca tcttccaggc acattgagag ccggagtctt 1920
ggagggagtt tagggtggtg attctacaac ggcgactagc aagtggcggg cttcagccct 1980
ttcccgctgc tctcctggtc gcgaccacac gtcacagctc tcgctcgttc cggttgctcg 2040
cgcagggggt ggggagtgtt gttaaccgga gcggctgccg cagtcgcggt gattgagcgt 2100
actccgccgc gccccgcgcc gccggaagtg aggtgtctta cccccgaagt tccggttcgc 2160
agggggtggg gagtgttgtt aaccggagcg gctgccgcag tcgcggtgat tgagcgtgct 2220
cgcggcgctg ggctcctggt gagtgggcct ggtcctgatt ggggttgggg ggtcggcgtc 2280
taggaccttg tcctttgggg tcactgcgat cagcccgccc cgctgcgttc ggccgccagt 2340
tttcggcctg tcagatggct ggagacctta ggcggcggcg cggccaccgt tccagaggcc 2400
gggccccgcc tgcgaggttc gcaactccta gcgttcacag gtgcgcgact gtgaggcgac 2460
ctgactggtt ctcagccccg ccgccgcacc ctggcggtcg gccgtttctc cggttctcag 2520
agtggacact gctgggggcg gggggggggg cagggttcca gactgacgta ccccgatggg 2580
cgcgcgtctg cgctgaccac cctggcacag ctgtcactgg ttgtgtcgcc ttctcaagct 2640
gtgccctctg caccttgcct cctccacccc tggcgggccc agcgaacctg cctctaaagc 2700
ctatcatccc agctccttca gagggtcagc ggtggcagcc cccctcctcc taactttgcc 2760
tcagtgactc cctagaggag gcgccttggc agacagcgtg gaagagccct agatttgaaa 2820

cgagattgat ccaagttcta ggccttgcat cagtgtgagc ctctaacccc tttgagtcct 2880

agtttctcgt ttgtgaaaca gggagtatat gctgttttga atctaatggc tgtcaaggtg 2940

aaatgagtgt ttgcccttac actctgccag ggactgtgct aggtttacat agtgtggata 3000

tcacaaatgt cattttcctt gtgcaggtct ctgggccagg gcgatg 3046

<210> 2
<211> 3092
<212> DNA
<213> Homo sapiens

<400> 2
ttggctcata acctcacttc ctttaagtct ttgctcaaat gtcaccttct caaggaagct 60

tacccgatta tcctcgctga tactgcaacc agcttcaagt accccaccac atcctgatcc 120

cctttattct gttctacttt tttcctatag cactgatcat cttccagcgt attagatttt 180

tcacttatgt ctgtggtttg ctgtcacatc tactaggata agctccacaa aggtagagat 240

ctttattttg ttcactgaca tcctaagtcc ctagaacagg agacacttga tccatatttg 300

tagactaact gaataaatga cttaattacc agtttggatg tgggggcaga tagtgagcat 360

gatgcccgtt tccggagctg gggtgcagac agtgtctagg gacactgaac tgttttaaaa 420

gcaggataga tcccggctgg agaccacaca aggaaatcat cagcacctgg gtcaggggct 480

ggactggagc agaggaaatc atgcaggaaa agtaaagaga aggacatcag gtaaagagaa 540

gaggacacat gcatagccag agagaaaaga ggagcagagg catgtggatc acagaagctt 600

agggaggaga ctttcaagaa ggggagagag gttgagtcaa gcaagggctg aaagccaacc 660

attggatgca gtcactagaa agttacagat aggcaaggtg ttgtggctca cgcctgtaat 720

cccaacacct tgtggggctg aggtgggagg atcgcttgag cccgggaggt cgaggctgca 780

atgagccctg atggcgccaa tgcactccag cctgggcgac agagcaagac cctgtcgcaa 840

aaattaataa ataaataaat aaaaagaaaa gggggaaaaa aagttatacg tggccttacg 900

gggaagccaa ctctgactgg ttataagctg aaactgtcaa gtcaacaggt ggcagggaag 960

atggctgaga ccaacagcac agagatttag aggcagacag acctggcgcc aatcctagga 1020

caggttttgg taagcctttg aatttcaatt gccccacgtt tcgggggagg gggtagcacc 1080

ccctagctca taaaccttag tgattgatga ttaaatgaga tgacggagga aaacgcaagg 1140

cacaaagtgg atgcattagc tccattttgt taatcagcag gcttagttgg ctgcgaccca 1200

gacacgaact aaaatacagt gcagcccagg accagtgggg gtcttgctta tggctcagag 1260

ctgaacaaca catgggcagc aaaatcagac actgagatgc gggcaggcct gcgacgctga 1320

agtcaattcc tttgaacaaa cagaacactt ccgtcccaag attagcagga attaatctcc 1380

cagtctcggg tacacctggt tgtccctccc tgtcctggcg cggcaaacgt tcccggaggc 1440

cagccaggga tcactcgccc aaggactgag ctttccctac tctcagccaa ctggagcggg 1500

accagggcct aggcaacgca gctgtccgcc cctaacaacc actcacctgc tttccccttt 1560

ctataggcca gcaaaggtac attctttttc ttattgggcc gcgtaactta tcgcaaccaa 1620

tcagtggcag ccacgggacc caactcactc ccacacaact tgtgggggtg atcatggaga 1680

agacaaattt ttgttttccg catccagttc tctcagagag caccgtattt gtcaaactgt 1740

tgtgactctc cctaaatgtt taagaaaaca tttcattccc ctcaggcttg tatagtctgt 1800

ccctggccta ctccccgctc caggtggtac agcccgcaag cggctcccct tcccagctgc 1860

tcgcggggcc gagtccccca gtccgaggag gccactcagc gcaggagcca taccatctgt 1920

gactaataaa taataggggg acctccgact cccccctgtt gccttattac cttccgacca 1980

cctctcggac ctcttgccca gcccttcccc gtagacatca ccccagatac ggtggtgaca 2040

ccattgctat gggcccacgt agggcgcagt gcgagccagg gcaggacgca cttggtacga 2100

cccacgccgc gccccgcgcc gccggaagtg aggtgtctga cccccgaagt tccggttcgc 2160

agggggtggg gagtgttgtt aaccggaggg gcagccgcag tcgcgcggat tgagcgggct 2220

cgcggcgctg ggttcctggt gagtggggcg aagtctggcc cgagttgtgg ttggggtcgg 2280

gacccgaacc ttccccttga ggtctccgga gtcggcacgc ccctcagccc cgccgcacgc 2340

tttcggcctg tcagctggcc ggagacctca gacgccggtg cggccgcttt gctcaagcct 2400

gggccctgcc tgcgacgccc gcaactcctg gtgctcacag gtgcgcggcc gcgagggcga 2460

cccggctcct cccgtcccgc tgctgctctc tcccgtcccg ctgtttttgt ggtgctctga 2520

gttgacacta ctccgggggt cgggggaccc caggattcca ggctgacgtt ccccgcccgc 2580

tcccgcaggg cgggcgtccg aactgcccac cctaacacag ctgtcaccgg cgctgtcgcc 2640

tgcccagcct gctatcctct gtgccttggc tgctctcagc cctggctgcg cattcccgcc 2700

cctggagcag atttctgctg ttgcctccca ccccatcttc tccaccggag ggtcagcggt 2760

gcagctcccc ctcctccaac attgcagctt ttcctcatca cctccctaga ggaggcggct 2820

tggcaggcag cgtggaaaga gccctagatt tgaagcaaga ctgacccagg ttccaggcct 2880

tgcgtcagtg tgatcactta accccttcga gtctaatttg taaaatgggg tagcgtaagc 2940

tattctttgt ctgatgattt cgagggcgaa atgtgatttc ccccccactt tctcctatga 3000

attgaggctg tgccaggcac cgggctattt tgcacagcac gagcatcaca taagttattt 3060

tcttgcccca tgcaggtctc cgggccaggg ca 3092

<210> 3
<211> 19

<212>  DNA
<213>  Artificial

<220>
<223>  synthetic DNA

<400>  3
gcgccgccgt aagtgaggt                                                    19


<210>  4
<211>  20
<212>  DNA
<213>  Artificial

<220>
<223>  synthetic DNA

<400>  4
aagtgagttg tcttaccccc                                                   20


<210>  5
<211>  20
<212>  DNA
<213>  Artificial

<220>
<223>  synthetic DNA

<400>  5
actccgccaa gccccgcgcc                                                   20


<210>  6
<211>  20
<212>  DNA
<213>  Artificial

<220>
<223>  synthetic DNA

<400>  6
gcgccgccgg aagtga                                                       16


<210>  7
<211>  20
<212>  DNA
<213>  Artificial

<220>
<223>  synthetic DNA

<400>  7
gcgccgccgt aagtga                                                       16

## Description

### TECHNICAL FIELD

The present invention relates to a promoter of the synoviolin gene.

### BACKGROUND ART

Synoviolin is a protein that was cloned from synoviocytes library of patients with rheumatoid arthritis by immunoscreening using anti-synovial cell antibodies.

Synoviolin is a ubiquitin ligase (E3) having a ring finger domain, and has a quality control function in the endoplasmic reticulum (ER). The amino acid sequence (nucleotide sequence) of synoviolin (the synoviolin gene) is highly homologous with yeast Hrd1, and has been shown to be the same molecule as human hrd1, which was cloned as a human homolog of yeast Hrd1 (Kaneko, FEBS Lett., Dec. 4, 532 (1-2): 147-52, 2002).

The following three mechanisms for quality control in the endoplasmic reticulum have already been discovered. One mechanism involves PERK (endoplasmic reticulum resident kinase). This mechanism regulates translation and reduces ER stress (explained below) by phosphorylating the translation initiation factor elf. The second is a mechanism of translation regulation via the ATF6 and XBP1 transcriptional factors by means of ATF6, IRE-1 and other ER transmembrane proteins that sense ER stress. In this mechanism, ATF6 and XBP1 induce transcription of Bip and other chaperone molecules, promoting a re-folding reaction of defective proteins. This reaction is called the UPR (unfolded protein response). The third is ERAD (ER-associated degradation), which reduces ER stress by breaking down proteins (Kaufman, RJ, Nat. Rev. Mol. Cell Biol., June, 3(6): 411-21, 2002). ERAD can be explained in detail as follows.

A protein which has been synthesized in the cytoplasm can only function when it has formed its proper three-dimensional structure and been transported to its assigned place. An unfolded or damaged protein which has not assumed an appropriate higher structure is checked by the quality control function of the cell, and is regenerated or broken down to maintain normal cell function.

Because proteins are unstable during the course of biosynthesis inside the endoplasmic reticulum lumen, they are exposed to various kinds of physical and chemical stress (including ischemia, hypoxia, heat shock, amino acid starvation, genetic mutation and the like). This kind of stress, called endoplasmic reticulum stress (ER stress), increases the occurrence of proteins having abnormal folding structures in the endoplasmic reticulum (unfolded proteins). Because defective proteins with three-dimensional structure abnormalities are not transported from the endoplasmic reticulum to the Golgi apparatus, unfolded proteins accumulate in the endoplasmic reticulum. In response to such ER stress, therefore, the cell breaks down the defective proteins by means of the endoplasmic reticulum-specific stress response mechanisms called UPR and ERAD, thus preventing stress to the ER from accumulation of such defective proteins. Synoviolin is induced by ER stress and is involved in the third mechanism, ERAD (Kaneko, FEBS Lett., Dec. 4, 532 (1-2): 147-52, 2002).

The inventors have also shown that increased expression of synoviolin suppresses sensitivity to apoptosis due to ER stress by promoting ERAD, and apoptosis sensitivity increases when synoviolin expression is lowered.

It has also been confirmed that expression is greater in rheumatic synovial cells than in normal synovial membrane. To verify the significance of synoviolin in arthritis, CIA (collagen induced arthritis: a mouse model in which bovine type II collagen is injected subcutaneously to immunize mice and cause arthritis by means of the resulting antibodies) was induced in a synoviolin hetero-knockout mouse. As a result, sensitivity to apoptosis due to ER stress was lower than in wild type mice, indicating CIA resistance. This seems to indicate that the requirements for expression are cell- and tissue-specific.

Based on these findings, since the mechanisms which regulate synoviolin levels control sensitivity to apoptosis in response to ER stress in cells, the elucidation of the mechanism is quite important.

### DISCLOSURE OF THE INVENTION

It is an object of the present invention to provide a promoter of the synoviolin gene.

The inventors completed the present invention as a result of exhaustive research aimed at resolving the aforementioned problems when they cleaved the synoviolin promoter region and, after investigating promoter activity in fragments of various lengths, succeeded in identifying the core region and elucidating its interactions with transcriptional factors.

That is, the present invention is as follows.
(1) A promoter of the synoviolin gene, comprising at least the nucleotide sequence of nucleotides 2120-2130 in the nucleotide sequence represented by SEQ ID: NO 1 or 2.
(2) A promoter of the synoviolin gene, comprising any nucleotide sequence selected from a group consisting of at least the nucleotide sequences of the regions of nucleotides 1-2201, 969-2201, 1142-2201, 1699-2201, 1880-2201, 2002-2201, 2094-2201 and 2118-2201 in the nucleotide sequence represented by SEQ ID: NO 1 or 2.
(3) A promoter of the synoviolin gene, comprising any nucleotide sequence selected from a group consisting of at least the nucleotide sequences of the regions of nucleotides No. 1-3043, 969-3043, 1142-3043, 1699-3043, 1880-3043, 2002-3043, 2094-3043 and 2118-3043 in the nucleotide sequence represented by SEQ ID: NO 1.
(4) A promoter of the synoviolin gene, comprising any nucleotide sequence selected from a group consisting of at least the nucleotide sequences of the regions of nucleotides No. 1-3092, 969-3092, 1142-3092, 1699-3092, 1880-3092, 2002-3092, 2094-3092 and 2118-3092 in the nucleotide sequence represented by SEQ ID: NO 2.
(5) The promoter of (a) or (b) below:
   (a) A promoter of the synoviolin gene, comprising a nucleotide sequence having a deletion, substitution or insertion in some region of the nucleotide sequence of the promoter according to any one of (1) to (4) above, and having promoter activity;
   (b) A promoter of the synoviolin gene, comprising a nucleotide sequence which hybridizes under stringent conditions with a sequence complementary to the nucleotide sequence of the promoter according to any one of (1) to (4) above, and having promoter activity.
(6) A gene expression cassette comprising the promoter according to any one of
(1) to (5) above, a target gene for expression and a terminator.
(7) A recombinant vector comprising the gene expression cassette according to (6) above.
(8) A transformant comprising the recombinant vector according to (7) above.
(9) A method for regulating transcription activity wherein the activity of the promoter according to any one of (1) to (5) above is inhibited or enhanced.

Inhibiting or enhancing the aforementioned promoter activity means inhibiting or enhancing the binding activity of a transcriptional factor. An example of a transcriptional factor binding site is the Ets binding site. Examples of transcriptional factors include any selected from the group consisting of GABPa, GABPβ, a complex of GABPa and GABPβ, Ets1, Pea3, Tel and Fli-1 for example.

### BRIEF DESCRIPTION OF THE DRAWINGS

Figure 1a shows the activity of cleaved synoviolin promoters.
Figure 1b shows the activity of cleaved synoviolin promoters.
Figure 1c shows the Ets binding site and core region of a synoviolin promoter.
Figure 1d shows transcription activity with a mutation introduced into the promoter.
Figure 2a is a photograph showing the results of gel shift assay.
Figure 2b is a photograph showing the results of gel shift assay.
Figure 2c is a photograph showing the results of gel shift assay.
Figure 2d is a photograph showing the results of gel shift assay.
Figure 3a shows the transcription activity of a synoviolin promoter.
Figure 3b shows the transcription activity of a synoviolin promoter.
Figure 3c shows the transcription activity of a synoviolin promoter in NIH3T3 cells knocked out using RNAi.
Figure 4a is a structural diagram of plasmids having LacZ bound to 3k and 1k synoviolin promoters.
Figure 4b is a photograph showing expression of a synoviolin promoter in a mouse embryo.
Figure 4c is a photograph showing expression of a synoviolin promoter in a mouse embryo.
Figure 5 is a photograph showing expression of a synoviolin promoter in RA synovial membrane cells.
Figure 6a shows photographs of apoptosis in NIH3T3 cells in which synoviolin has been knocked down using RNAi, along with the results of western blotting.
Figure 6b shows photographs of apoptosis in NIH3T3 cells in which synoviolin has been knocked down using a decoy ODN, along with the results of western blotting.
Figure 7a is a photograph showing cells in which synoviolin is overexpressed.
Figure 7b is a photograph showing suppression of synoviolin expression by EBS decoy ODNs.
Figure 7c is a photograph showing apoptosis caused by EBS decoy ODNs treatment in NIH3T3 cells in which synoviolin is overexpressed.
Figure 7d shows apoptosis caused by EBS decoy ODNs treatment in NIH3T3 cells in which synoviolin is overexpressed.

### BEST MODE FOR CARRYING OUT THE INVENTION

The present invention is explained in detail below.

### 1. Outline

The inventors are presently analyzing pathogenesis of RA with a focus on synoviolin. We recently discovered from an analysis using a synoviolin knockout mouse that synoviolin levels which functions in ERAD are involved in threshold values of apoptosis induction in response to ER stress.

We therefore performed a promoter analysis to elucidate the transcriptional mechanisms regulating synoviolin levels, which determines the sensitivity of apoptosis induction.

At present, one mechanism responsible for inductive synoviolin gene expression is thought to be transcriptional regulation by means of XBP-1 spliced via IRE-1 which has been phosphorylated after sensing ER stress. This regulation mechanism is as follows. When unfolded proteins accumulate in the ER, the ER-localized membrane protein IRE-1 dimerizes and undergoes intermolecular self-phosphorylation. Endonuclease-like domains within IRE-1 are activated as a result and splice immature XBP-1 mRNA, producing mature XBP-1. XBP-1 is thought to promote transcription of ER molecule chaperone genes and the like.

However, there have been no reports on constitutive gene expression. We therefore began by using a mouse cell strain and mouse embryo to determine the site responsible for constitutive gene expression.

As a result, EBS was identified as being responsible for constitutive gene expression, and its element was shown to be essential for synoviolin expression in vivo. In NIH3T3 cells, it was shown that a member of the Ets family, the GABP a/β complex, is involved in transcriptional regulation via EBS.

Hetero-knockout (LacZ knock in mouse) expression analysis and northern and western analysis have shown that synoviolin is expressed ubiquitously. That is, it appears that synoviolin expression is necessary in many cells. This was deduced from the fact that the synoviolin knockout mouse died in embryo due to systemic apoptosis promotion. Expression may be stronger or weaker, however, and expression is particularly strong in cells with strong secretory ability (pancreas, testes, and nerve cells). This suggests that especially high synoviolin levels expression are required in some cells.

To understand the transcriptional regulation that determines synoviolin levels, we performed a promoter analysis in the present invention. Mechanisms of transcriptional regulation include (i) constitutive gene expression including basic transcriptional complexes and (ii) induced gene expression in response to a stimulus. It has been reported in the past that synoviolin is induced in response to ER stress, but there have been no reports on constitutive gene expression. The inventors therefore investigated the elements responsible for constitutive gene expression.

No TATA box or initiator sequence is present in the synoviolin promoter. In the case of such promoter structures, transcriptional factors of the SP 1 and Ets families and the like are important for inducing transcription.

The Ets family is a transcriptional factors having a domain called Ets which has been conserved from yeasts to humans. This family has more than 30 members, most of which are involved in transcriptional activation of molecules responsible for differentiation, proliferation and apoptosis (D.K. Watson and A. Seth, Oncogene review issue, Dec. 18, 19(55), 2000).

Of this Ets family, the transcriptional factor called GABPa has certain features related to transcriptional regulation of target genes. First, GABPa has DNA binding ability due to its Ets domain but no transcriptional activation ability. By contrast, the GABPa cofactor GABPβ has no DNA binding ability but induces transcriptional activity when it forms a dimer with GABPa, and acquires even higher transcriptional activation ability when it forms a hetero-tetramer (Yu, M., J. Biol. Chem. Nov. 14, 272(46), 29060-7, 1997). Moreover, GABPa can function as an initiator in expression of genes having no TATA box or genes having multiple transcription initiation sites (Yu, M., J. Biol. Chem. Nov. 14, 272(46): 29060-7, 1997; Kamura, T., J. Biol. Chem. Apr. 25; 272(17): 11361-8, 1997). Although GABPa is expressed ubiquitously, it acts synergistically with partner transcriptional factors binding to other sites to control expression of genes involved in cell-specific differentiation and proliferation (Schaeffer, L, EMBO J. Jun. 1; 17(11): 3078-90, 1998).

The inventors therefore determined the elements responsible for constitutive gene expression of synoviolin and investigated the significance of those elements in NIH3T3 cells.

### 2. Promoter

A promoter region can be obtained by cleavage using a restriction enzyme from the synoviolin gene or a mouse or human genome sequence or the like. In general, however, there may not be a convenient restriction enzyme site located at a suitable position. The desired promoter region can therefore be obtained by PCR amplification using primers that already have restriction enzyme recognition sites. A desired promoter region can also be chemically synthesized based on the nucleotide sequence data for a known promoter region.

### (1) Promoter sequence

The total promoter sequence of the mouse synoviolin gene is shown by SEQ ID: NO 1, and the total promoter sequence of the human synoviolin gene by SEQ ID: NO 2, but the promoter sequence is not limited to these as long as it has actual transcription activity, the full length promoter can be cleaved and the nucleotide sequence can also be mutated by a deletion, insertion, substitution, addition or the like in part of its sequence. However, because in the present invention introduction of a mutation into the core region (comprising the Ets binding site) is likely to cause a diminution in promoter activity, mutations into the core region are preferably introduced when the aim is to suppress promoter activity.

Normal site-directed mutagenesis methods can be adopted for introducing gene mutations, and a Gene Tailor™ Site-Directed Mutagenesis System (Invitrogen), TaKaRa Site-Directed Mutagenesis System (Mutan-K, Mutan-Super Express Km: Takara Bio) or the like can be used for example.

In the promoter of the present invention, the core region which is central to its function is the region of the nucleotide sequence shown by SEQ ID: NO 1 or 2 from nucleotides 71 (-71) to 81 (-81) moving upstream (in the 5' direction) from the transcription initiation site (t of position 2201 in the nucleotide sequence shown by SEQ ID: NO 1 or 2) as the starting point, or in other words the region of nucleotides 2120 to 2130 in the nucleotide sequence shown by SEQ ID: NO 1 or 2.

Consequently, the length is not restricted as long as the aforementioned core region is included of the total sequence shown by SEQ ID: NO 1 or 2. For example, the full-length and cleaved sequences shown in Tables 1 and 2 below (see example 1), which include the aforementioned core sequence, are included in the promoter of the present invention. Moreover, a sequence which hybridizes under stringent condition with a sequence complementary to a sequence comprising the aforementioned core sequence in the promoter sequence represented by SEQ ID: NO 1 or 2 or a cleaved promoter sequence thereof, and which has synoviolin promoter activity, is included in the promoter of the present invention.

In the present invention, when the location of a promoter region is indicated it may be specified either based on the nucleotide numbers of SEQ ID: NO 1 or 2 or based on the translation initiation site. When the translation initiation site is specified, the translation initiation site (TS) is the thymidine (t) of position 2201 in the nucleotide sequence of SEQ ID: NO 1 or 2. Starting at the TS, upstream (5') positions are indicated with negative (-) numbers and downstream (3') positions with positive numbers (+). In the 5' direction, the nucleotide 1 nucleotide upstream (5' direction) from the TS is designated -1, while in the 3' direction the TS nucleotide is designated + 1. For example, the first guanine (g) of the nucleotide sequence represented by SEQ ID: NO 1 is in the -2201 position starting from the TS, while the 3043^{rd} guanine (g) in the nucleotide sequence represented by SEQ ID: NO 1 is in the +843 position starting from the TS.

"Stringent conditions" mean conditions in which the salt concentration for washing during hybridization is 100 to 500 mM or preferably 150 to 300 mM and the temperature is 50 to 70°C or preferably 55 to 65°C. "Promoter activity" means that transcription results when a transcriptional factor binds to the promoter region of the present invention.

DNA fragments which "hybridize under stringent conditions" include those which may hybridize under the aforementioned stringent conditions even if there is a deletion, replacement, insertion or addition to some region of the nucleotide sequence. Because the promoter occurs in cleaved forms of a variety of lengths there is no particular limit on the number of the aforementioned deletions, substitutions, insertions, additions and the like as long as promoter activity is retained, but an example would be a fragment having the aforementioned mutations in one or multiple (1 to about 10 or preferably 1 to about 5) nucleotides.

### (2) Construction of recombinant vector

The recombinant vector of the present invention can be obtained by inserting the promoter of the present invention, the target gene for expression and a terminator into an appropriate vector. This is called a "gene expression cassette". Examples of vectors used for gene expression cassettes include pBR, pUC, pBluescript and other *E. coli* plasmid vectors, pcDNA3 mammal cell expression plasmid vectors and adenoviruses, retroviruses and other virus vectors. The target gene for expression need not be the synoviolin gene, and may also be a heterologous gene (such as IL-1, IL-6, TNFa and other cytokine genes, enzyme genes, growth factor genes, chemokine genes and the like). A person skilled in the art can easily insert the constituent elements of the expression vector into the vector using well known techniques. Selection marker genes and the like can also be included in the aforementioned vector. Examples of selection marker genes include G-418 and other antibiotic resistance genes, and the thymidine kinase gene, diphtheria toxin gene and the like.

There are no particular limits on the terminators that can be used in the present invention, but examples include the poly A signal and the like.

### (3) Transformant

Common techniques used in transforming to hosts can be adopted for introducing the plasmid into the host. That is, the protoplast method, lithium method, electroporation method and modifications of these that are well known to those skilled in the art can be applied. There are no particular limitations on the host, and examples include *E*. *coli* and other bacteria, yeasts, animal cells, insect cells and the like.

### 3. Functional analysis of the promoter

### (1) Analysis of transcription activity

The transcription activity of the promoter can be analyzed by ordinary luciferase assay, gel shift assay, CAT assay or the like. Kits for performing these assays are commercially available (such as the Promega dual luciferase assay kit).

In the case of a luciferase assay for example, the firefly luciferase gene is attached as a reporter upstream from the transcription initiation site of the target gene. A vector having the cytomegalovirus (CMV) beta-galactosidase (β-gal) gene attached downstream from the promoter as a reporter can be introduced into the cell at the same time to correct the efficiency of introduction between cells which are the target of the assay. The calcium phosphate method for example can be adopted for introduction into cells. Cells with the introduced vector are collected after a fixed culture time and disrupted by freezing-liquefaction or the like, and luciferase and β-galactosidase activity are measured using a fixed amount of cell extract.

### (2) EMSA

Cell differentiation and activation is controlled by gene expression, and regulation of gene expression on the transcriptional level plays a particularly important role. It is becoming clear that most transcriptional factors bind in different ways to DNA response elements. One method of analyzing the complex relationships between these transcriptional factors is gel shift assay (EMSA: electrophoretic mobility shift assay). Gel shift assay is a method in which a radioactively or non-radioactively labeled probe DNA fragment is mixed with a DNA-binding protein, and electrophoresed with a non-denatured gel with a low salt concentration, and the DNA is detected by autoradiography or the like. Because DNA binding to the protein moves more slowly through the gel than free DNA, it can be detected as a specific band. When specific antibodies for the DNA-protein complex are applied, the rate of movement falls still further (called a supershift). Many transcriptional factors can be identified by this method.

In cells, however, some of the secretory proteins, membrane proteins and other proteins synthesized by way of the endoplasmic reticulum acquire defects in the endoplasmic reticulum, and breakdown of these defective proteins requires expression of molecules responsible for ERAD, which is an essential mechanism in the cell. This is clear from the fact that synoviolin knockout mice die in embryo, as shown in the examples below. That is, a minimum level of synoviolin expression is required by cells. The necessary site for this transcriptional regulation is the EBS (Ets binding site). Abnormalities in signaling via this EBS site act negatively on the maintenance of cell function. On the other hand, the members of the Ets family are diverse and expressed in diverse ways, and bind to the site synergistically with other partners. This means that the transcriptional regulation mechanisms of various members of the Ets family are involved in the EBS site.

Therefore, by promoting or inhibiting the action of a protein that binds to the EBS site it is possible to regulate the transcriptional activity function of the promoter of the present invention.

Examples of transcriptional factors include GABPa, GABPβ, a complex of GABPa and GABPβ, Ets1, Pea3, Tel and Fli-1 for example.

GABPa, a member of the Ets family having an Ets domain, is a protein consisting of 454 amino acids. GABPa is expressed ubiquitously in cells. It forms a hetero-dimer with GABPβ via the Ets domain. It is also known to form a hetero-tetramer using two Ets binding sites.

GABPβ is a cofactor of GABPa having 382 amino acids. It is not a member of the Ets family. It forms a hetero-dimer with GABPa via the Ankyrin repeat sequence. It has a transcription activity domain.

Ets 1 is a protein having 441 amino acids, and is a human homolog of v-ets, which was discovered in 1983 as a proto-oncogene of the retrovirus E26, which causes erythroblastosis in chickens.

Tel is a protein having 452 amino acids, and has been reported to be a member of the Ets family which functions in suppressing transcription. Clinically, it is known to cause leukemia by forming a fused protein with AML1 by means of chromosomal translocation of t (12;21).

Fli-1 is known to cause Ewing's tumor by forming a fused protein with EWS by means of chromosomal translocation of t (11;22), and it has 452 amino acids.

The present invention is explained in more detail below using examples. However, the present invention is not limited by these examples.

### [Example 1] Preparation of synoviolin promoter

### (1) Plasmid construction

A roughly 7.5 kbp genome from the 5' to the 3' end comprising the synoviolin gene was subcloned from a mouse (C57B1/6) genome into SyB/pBluescript. Next, the promoter region of the synoviolin gene was treated with XhoI and NcoI, and a roughly 3k fragment was removed and inserted (SyG-2.2k) into PGV-B2 (TOYO INK GROUP). This roughly 3k fragment was the full-length promoter (SEQ ID: NO 1). Certain regions were also deleted from the 5' end of the full-length promoter to prepare constructs with abbreviated promoter regions. The resulting promoters can be summarized as follows.

**Table 1 Table of Prepared Promoters (Mouse)**

| Name | Region* | Region of nucleotide sequence shown by SEQ ID: NO 1 |
|---|---|---|
| -2201/+843 (full-length) | Region of 2201 nucleotides upstream (5') and 843 nucleotides downstream (3') starting at TS (translation initiation site: +1) | 1-3043 |
| -1233/+843 | Region of 1233 nucleotides upstream and 843 nucleotides downstream starting at TS | 969-3043 |
| -1060/+843 | Region of 1060 nucleotides upstream and 843 nucleotides downstream starting at TS | 1142-3043 |
| -503/+843 | Region of 503 nucleotides upstream and 843 nucleotides downstream starting at TS | 1699-3043 |
| -322/+843 | Region of 322 nucleotides upstream and 843 nucleotides downstream starting at TS | 1880-3043 |
| -200/+843 | Region of 200 nucleotides upstream and 843 nucleotides downstream starting at TS | 2002-3043 |
| -108/+843 | Region of 108 nucleotides upstream and 843 nucleotides downstream starting at TS | 2094-3043 |
| -84/+843 | Region of 84 nucleotides upstream and 843 nucleotides downstream starting at TS | 2118-3043 |
| -73/+843 | Region of 73 nucleotides upstream and 843 nucleotides downstream starting at TS | 2129-3043 |
| -65/+843 | Region of 65 nucleotides upstream and 843 nucleotides downstream starting at TS | 2137-3043 |
| -39/+843 | Region of 39 nucleotides upstream and 843 nucleotides downstream starting at TS | 2163-3043 |
| -10/+843 | Region of 10 nucleotides upstream and 843 nucleotides downstream starting at TS | 2191-3043 |

| | | |
|---|---|---|
| *The first nucleotide on the 5' side of the TS (t #2201 in SEQ ID: NO 1) is counted as -1 moving upstream (toward 5'), while the TS is counted as +1 moving upstream (toward 3'). | | |

Cleaved promoters of the human synoviolin gene can be prepared in the same way as for mice. The locations of the cleavage sites in this case are shown in Table 2.

**Table 2 Table of Prepared Promoters (Human)**

| Name | Region* | Region of nucleotide sequence shown by SEQ ID: NO 2 |
|---|---|---|
| -2201/+892 (full-length) | Region of 2201 nucleotides upstream (5') and 892 nucleotides downstream (3') starting at the TS (translation initiation site: + 1) | 1-3092 |
| -1233/+892 | Region of 1233 nucleotides upstream and 892 nucleotides downstream starting at TS | 969-3092 |
| -1060/+892 | Region of 1060 nucleotides upstream and 892 nucleotides downstream starting at TS | 1142-3092 |
| -503/+892 | Region of 503 nucleotides upstream and 892 nucleotides downstream starting at TS | 1699-3092 |
| -322/+892 | Region of 322 nucleotides upstream and 892 nucleotides downstream starting at TS | 1880-3092 |
| -200/+892 | Region of 200 nucleotides upstream and 892 nucleotides downstream starting at TS | 2002-3092 |
| -108/+892 | Region of 108 nucleotides upstream and 892 nucleotides downstream starting at TS | 2094-3092 |
| -84/+892 | Region of 84 nucleotides upstream and 892 nucleotides downstream starting at TS | 2118-3092 |
| -73/+892 | Region of 73 nucleotides upstream and 892 nucleotides downstream starting at TS | 2129-3092 |
| -65/+892 | Region of 65 nucleotides upstream and 892 nucleotides downstream starting at TS | 2137-3092 |
| -39/+892 | Region of 39 nucleotides upstream and 892 nucleotides downstream starting at TS | 2163-3092 |
| -10/+892 | Region of 10 nucleotides upstream and 892 nucleotides downstream starting at TS | 2191-3092 |

| | | |
|---|---|---|
| *The first nucleotide on the 5' side of the TS (t #2201 in SEQ ID: NO 1) is counted as -1 moving upstream (toward 5'), while the TS is counted as +1 moving upstream (toward 3'). | | |

Next, SyG-2.2kG-76T/BV2 was prepared using site-directed mutagenesis by overlap extension (Molecular cloning, CSHL Press, 3^{rd} Edition, 2001, Chapter 13) for purposes of preparing mutants of the aforementioned mouse-derived promoters. The following primers were used.
1. EBSm (G-76T): GCGCCGCCGTAAGTGAGGT (SEQ ID: NO 3)
2. AMLm (G-68T): AAGTGAGTTGTCTTACCCCC (SEQ ID: NO 4)
3. SP1m (G-92A, C-91A): ACTCCGCCAAGCCCCGCGCC (SEQ ID: NO 5)

PCR was performed under the following conditions using a reaction composition comprising 1 pmol SyB/pBluescript, 100 pmol primer, 0.2 mM dNTPs, 5U polymerase, 10 mM Tris-HCl (pH 8.3) and 50 mM KCl in 50 µl of reaction solution.

### PCR conditions:

First stage: 94°C, 1 minute → (94°C, 30 sec → 55°C, 30 sec → 72°C, 1 min) x 25 times
Second stage: 1^{st} cycle: 94°C, 1 min → 55°C, 30 sec → lowered to 30°C over 29 minutes → 30°C, 1 min → to 72°C over 9 minutes → 72°C, 1 min → (94°C, 30 sec → 55°C, 30 sec → 72°C, 1 min) x 25 times

EBSm (G-76T) is a primer for mutating G to T in the 2125^{th} position (76^{th} (-76) upstream from the TS) in the nucleotide sequence shown by SEQ ID: NO 1, AMLm (G-68T) is a primer for mutating G to T in the 2133^{rd} position (68^{th} (-68) upstream from the TS) of the nucleotide sequence shown by SEQ ID: NO 1, SP1m (G-92A) is a primer for mutating G to A in the 2111^{th} position (92^{nd} (-92) upstream from the TS) in the nucleotide sequence shown by SEQ ID: NO 1, and SP1m (G-92A, C-91A) is a primer for mutating C to A in the 2112nd position (-91 from TS) in the nucleotide sequence shown by SEQ ID: NO 1 and mutating G to A in the 2111st position (-92 from TS) of the nucleotide sequence shown by SEQ ID: NO 1).

### [Example 2] Functional analysis of synoviolin promoter

A promoter analysis was performed to elucidate the mechanisms regulating synoviolin levels. Synoviolin has been shown from transgenic mouse (LacZ knock in) analysis and northern and western results to be expressed ubiquitously.

After the synoviolin promoter had been cloned, a region comprising 2.2k from the translation initiation site was bound to a luciferase vector. The transcription activity of various constructions cut from the upstream side was investigated using a variety of cells (shown below). The cells were cultured using DMEM medium (Life Technologies, Inc.) with 10% added inactivated fetal cow serum.

Cells used:
ATDC5: Sub-strain derived from mouse teratocarcinoma AT805 cells; cartilage and chromatophore specific; alkaliphosphatase positive
HEK293: Human fetal kidney-derived cells
NIH3T3: Mouse fetus-derived fibroblasts
Transfection and reporter assay were performed as follows.

The cells were prepared 2 x 10⁴/well on 24-well plates. 24 hours later, they were transfected (Biochem.Roche) using a FUGENE 6 kit in accordance with the attached manual. 50 ng each of CMV-β-gal was used to correct the transfection efficiency, and the total vector alone was added to a total of 150 ng.

The protein was collected 30 hours after transfection. The medium was discarded, and after washing with PBS the cell lysate was collected using 100 µl of Passive dissolution buffer (Promega). Next, 20 µl of this cell lysate was transferred to a 96-well plate, and luciferase activity was measured with a luminometer. β-gal activity was also measured with a plate reader, and the Luc value was then corrected by dividing with the β-gal value. β-gal staining was done as follows. Using X-gal as the β-galactosidase, stained (5-bromo-4-chloro-3-indolyl- β- D-galactopyranoside; Sigma). Embryos were fixed for 20 minutes in 4% paraformaldehyde, soaked in X-gal solution, and stained for 12 to 24 hours at 37°C. After staining they were washed in PBS, and fixed again in 4% paraformaldehyde.

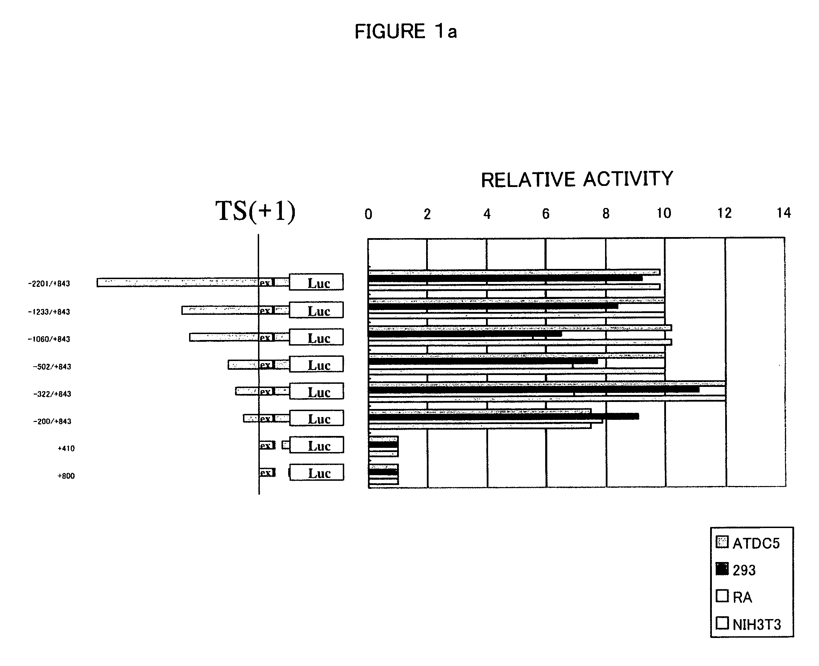

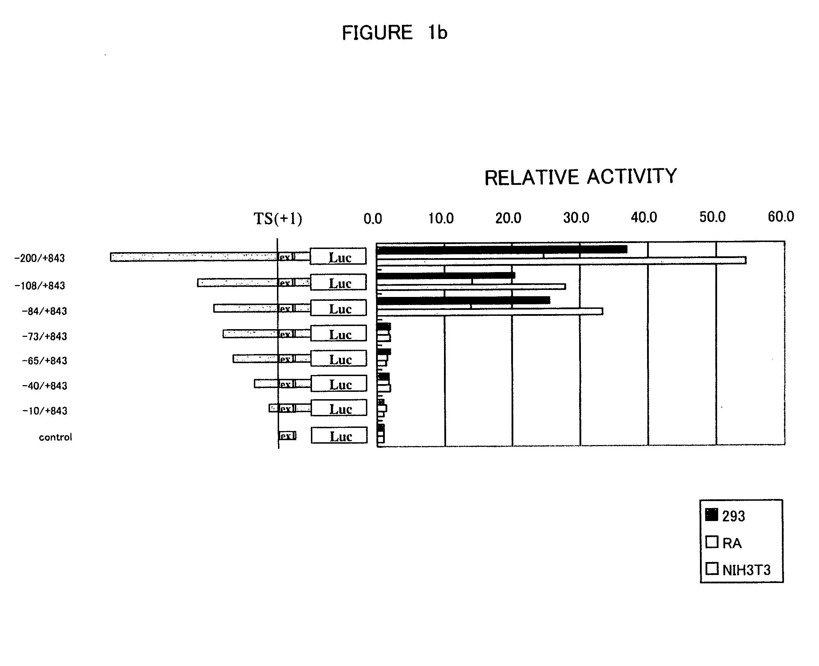

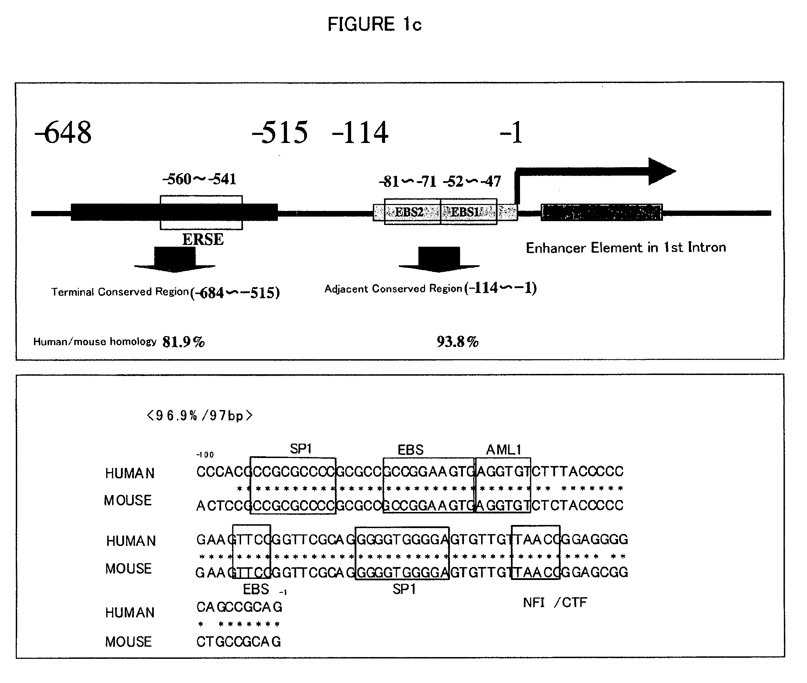

As a result, transcription activity was reduced by from 10 to 30% by deleting the region (12 nucleotides) from -84 to -73 bp of the promoter sequence (Figures 1a, 1b).

The region from -1 to -114 including the aforementioned deleted region is 94% homologous between mice and humans, while the 12 nucleotides are 100% homologous. Analysis using a computer with Bioinformatix analysis software (http://www.cbrc.jp/ research /db/TFSEARCHJ.html) showed the presence of the EBS (Ets binding site) in these 12 nucleotides (Figure 1c).

Next, the inventors introduced mutations into the transcriptional factor binding sequences of the previous and subsequent regions including that region, and investigated the effects on transcription of those sites using various cell lines.

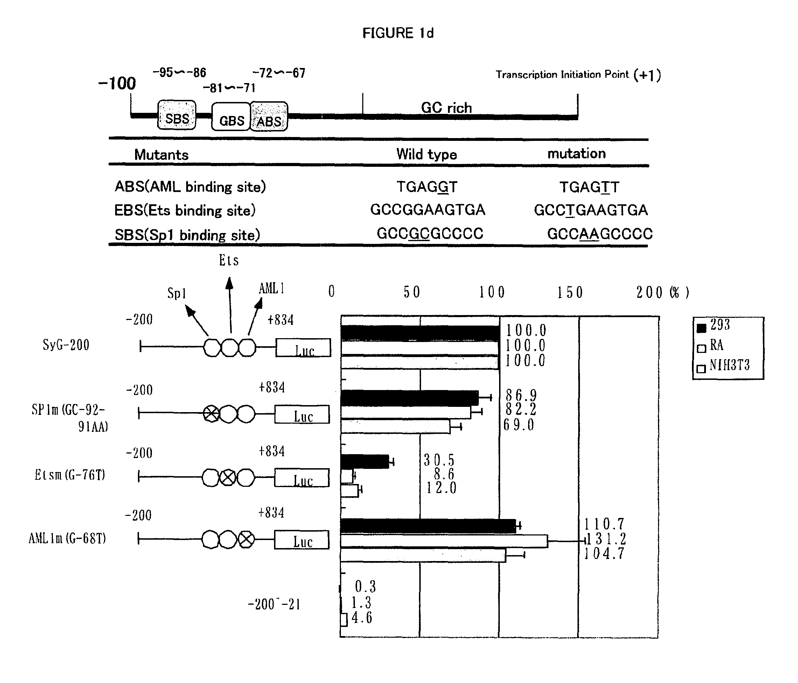

As shown in Figure 1d, activity was reduced by from about 9 to 40% in almost all cell lines by point mutations in the EBS (also called the GBS, shown as "GBS" in Figure 1d). This suggests that this EBS is an essential element for constitutive expression of synoviolin.

### [Example 3] Identification of transcriptional factors for EBS (from -85 to -70)

The Ets family is formed of at least 30 members all of which have the Ets domain, which is a DNA binding domain. Its central sequence is GGAA, and the sequence following that sequence is thought to be important for binding to the sequence.

First, probes comprising the 12-nucleotide sequence were used to investigate whether or not transcriptional factors actually bind to that sequence. Transcription factor binding was tested by gel shift assay (EMSA).

EMSA was performed as follows.

Probes:
EBS WT (-85 to-70): GCGCCGCCGGAAGTGA (SEQ ID: NO 6)
EBS MT (-85 to -70): GCGCCGCCGTAAGTGA (SEQ ID: NO 7)

The EMSA probes were prepared by annealing sense chains and antisense chains with 25-nucleotide sequences for 10 minutes at 90°C.

Next, T4 nuclease kinase, buffer and ?³²P were mixed and the mixture was reacted for 30 minutes at room temperature. The reaction solution was passed through a Micro Spin G-25 column (Amersham Pharmacia Biotech), and the collected column fraction was centrifuged to obtain labeled probes. Those having radioactivity of 30000 cpm or more per 1 µl were used.

10 µg of protein was mixed with reaction buffer and probe, and reacted for 30 minutes at room temperature. For purposes of supershift, they were reacted for 1 hour at 4°C before being reacted with the probe. The reaction solution was electrophoresed for about 3 hours at 100v, 50 mA in non-denatured gel. The gel was dried, and analyzed by autoradiography using a Fujii BAS2000.

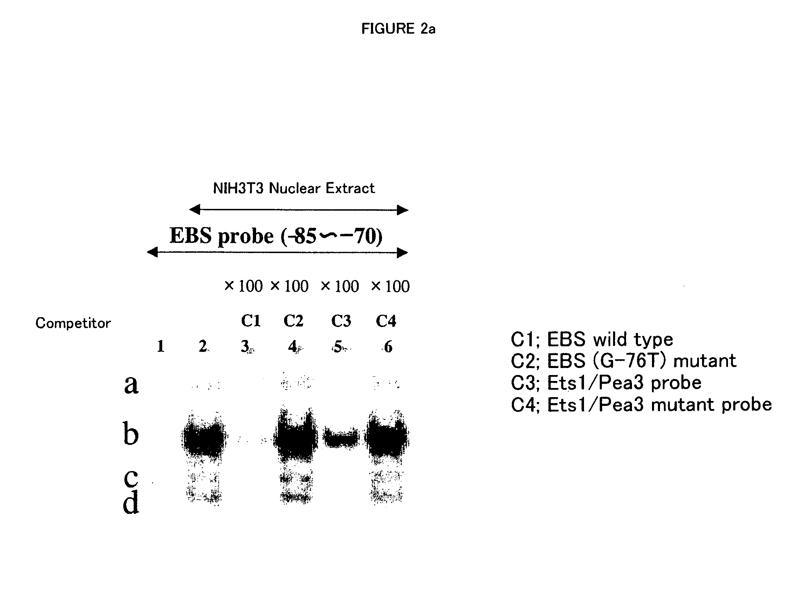

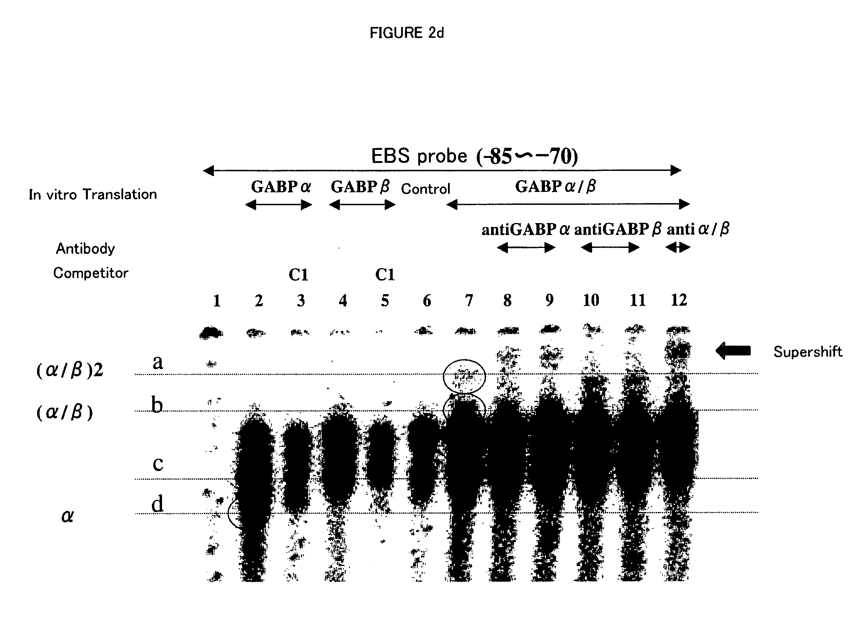

As a result, 4 bands (bands at locations a, b, c and d in Figure 2a) were formed by gel shift using NIH3T3 nuclear extract. In cold competition (competitive testing using unlabeled probe), all bands disappeared, and no interference was seen with a probe having a single-nucleotide mutation (Figure 2a).

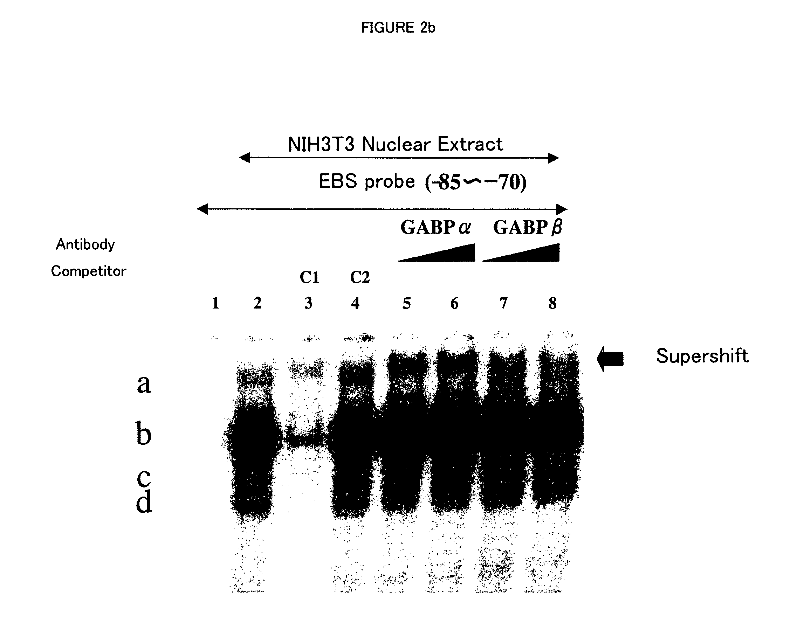

When cold competition was attempted using Ets1/Pea3 and Ets probes to see what Ets family members would bind to the promoter sequence, competitive interference occurred with the ets1/pea3 probe, and interference was eliminated by mutation (lanes 5 and 6 in Figure 2b).

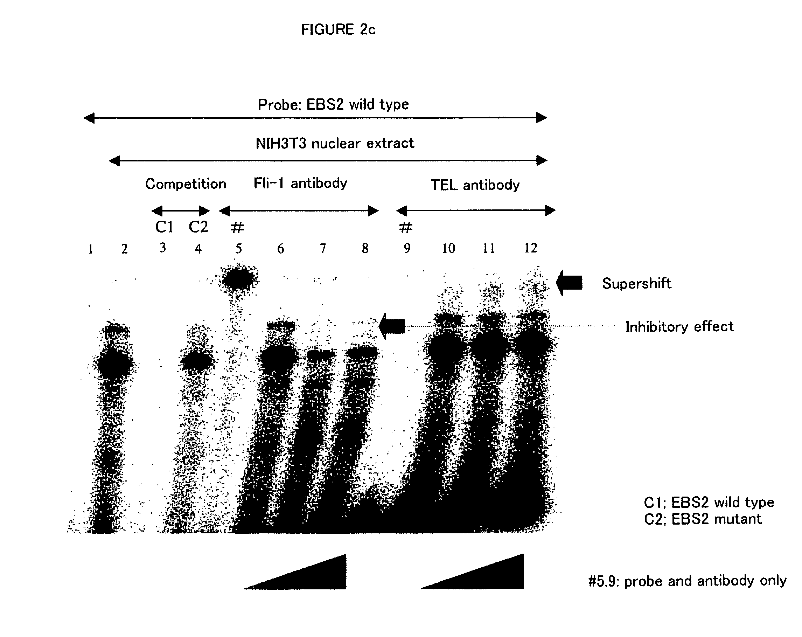

This suggests that the factors that bind to the promoter sequence may be the Ets 1, Pea 3, GABPa and other transcriptional factors which are factors which bind to the Ets 1/Pea3 probe (purchased from Santa Cruz Co.). In supershift testing using a variety of antibodies, GABPa and Tel supershifted while Fli-1 exhibited an interference effect (lanes 5 and 6 in Figure 2b, lanes 6, 7 and 8 and 10, 11 and 12 in Figure 2c). When a gel shift test was performed using the in vitro translation products of GABPa, Fli-1 and ets1, all exhibited a supershift. This indicates that multiple members of the Ets family bind to this sequence.

Next, complex formation with GABPβ was tested using supershifted GABPa. In gel shift testing using the respective in vitro translation products, a new band a,b (complex) was formed in lane 7, in which the GABPβ protein was added to the GABPa protein. Addition of GABPβ antibodies interfered with the formation of complex a (lanes 10 and 11 in Figure 2d).

These results show that GABPa/β form a complex on the EBS of the synoviolin promoter (Figure 2d).

### [Example 4] Transcriptional regulation by the Ets family in NIH3T3 and effects of GABPa, Fli-1 and Ets1 on synoviolin transcription activity

The synoviolin transcriptional activation ability in cells of transcriptional factors which bind to the EBS was evaluated by a transcriptional activation assay.

### (1) Oligo preparation of ODNs and preparation of cell extract

20-nucleotide oligodeoxyribonucleotides (ODNs) were obtained by chemical synthesis. A decoy ODN was also prepared by annealing of sense and antisense oligonucleotides. Cells in the logarithmic growth phase were treated with trypsin and transferred to 96-well plates (1 x 10³/well). After 24 hours, 20 pmol of decoy ODN was added to each well, and transfection was performed for 3 days using LipofectAMINE 2000 (Invitrogen, San Diego, CA) in accordance with the instructions. A cell proliferation assay was performed using Alamar Blue (Biosource International) in accordance with the kit's instructions to determine proliferation of cells having the decoy ODN.

### (2) Oligo preparation of RNAi and preparation of cell extract

21-nucleotide RNA was obtained by chemical synthesis. siRNA was prepared in accordance with the protocols of Elbashir et al (Elbashir, S.M., *Nature* 411: 494-498, 2001). Cells in the logarithmic growth phase were treated with trypsin and transferred to 96-well plates (1 x 10³/well). After 24 hours, 20 pmol of siRNA was added to each well, and transfection was performed for 3 days using LipofectAMINE 2000 (Invitrogen, San Diego, CA) in accordance with the instructions. A cell proliferation assay was performed using Alamar Blue (Biosource International) in accordance with the kit's instructions to determine proliferation of the siRNA cells.

### (3) Results

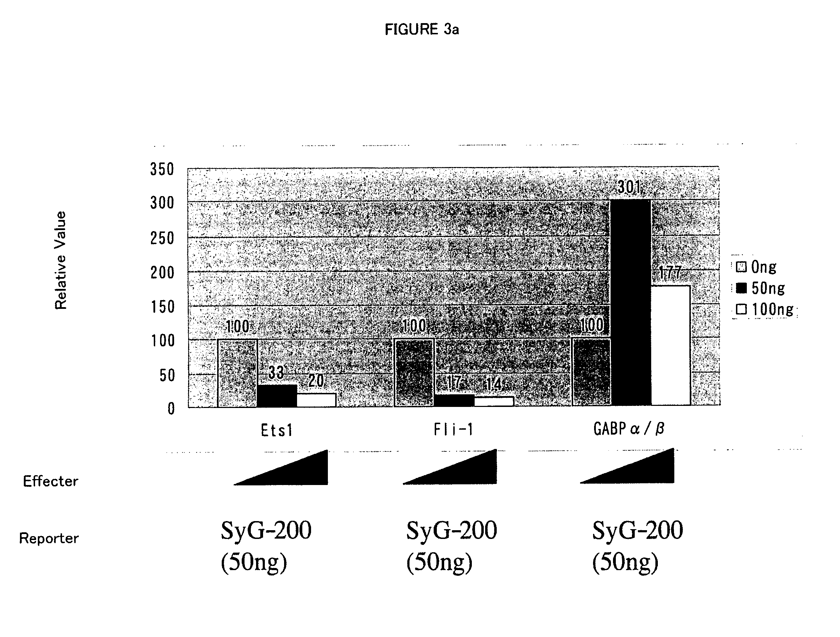

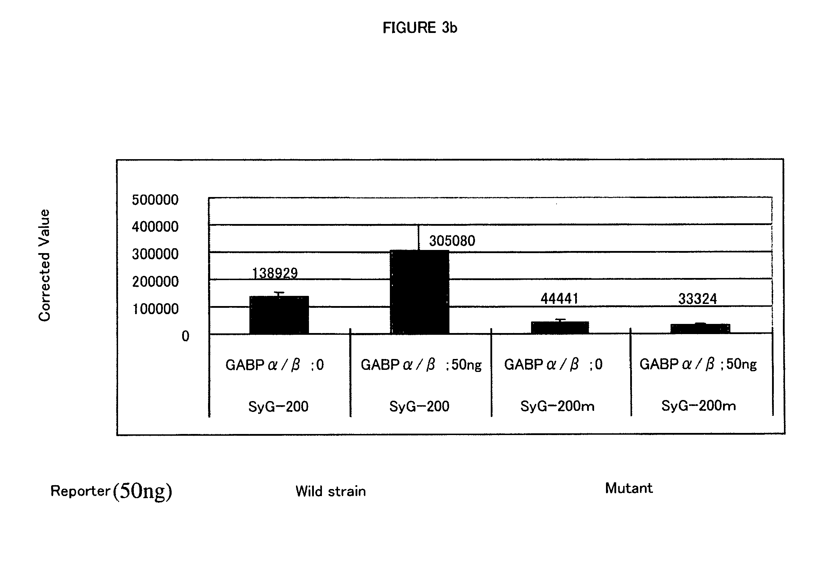

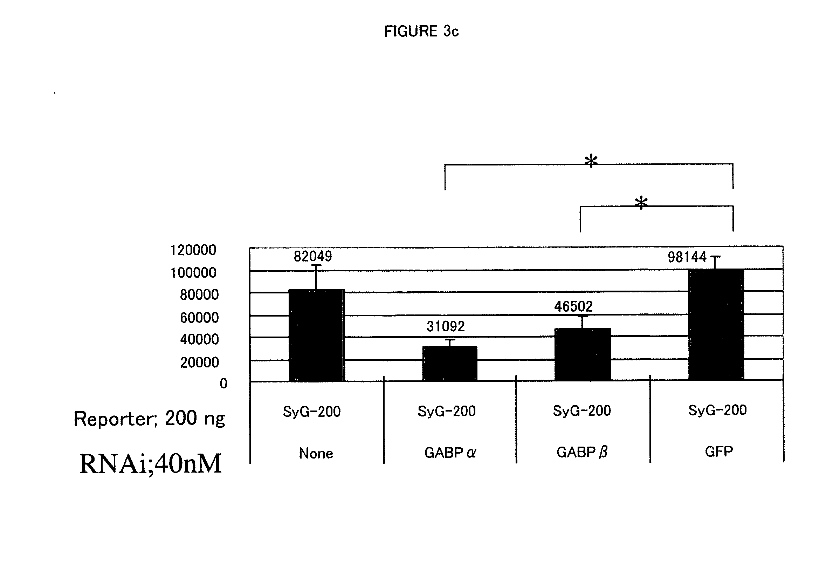

In NIH3T3, Fli-1 suppressed synoviolin transcription activity, while GABPa increased transcription activity (Figures 3a, 3b). Synoviolin transcription was also lower in NIH3T3 cells in which the GABPa and GABPβ RNAi was knocked out (Figure 3c). This suggests that GABPa/β is responsible for expression of synoviolin in NIH3T3 cells.

Next, to show that this GABPa/β complex is responsible for transcription activity of synoviolin via the EBS, a transcription activity assay was performed using promoters (-200 to +843) having an EBS (G-76T) mutant and the wild EBS, respectively.

As a result, transcriptional activation with the wild type was about three times that with the mutant even though the mutant was not activated at all.

### (4) Western blotting

Next, NIH3T3 cells were treated with 200 nM of decoy, and synoviolin expression was evaluated by western blotting.

Western blotting analysis was performed as follows. Cell culture was collected, and dissolved in a solution comprising 1% NP-40, 25 mM Tris-HCI, pH 6.8, 0.25 % SDS,0.05% 2-mercaptoethanol and 0.1 % glycerol. An aliquot of the clear cell lysate was isolated on SDS-polyacrylamide gel. The isolated protein was transferred to a nitrocellulose membrane, and immunoblotted using anti-synoviolin monoclonal antibodies. The bound antibodies were detected with peroxidase-binding goat anti-mouse immunoglobulin and an ECL detection system (Amersham Pharmacia Biotech).

As a result, expression of synoviolin was reduced by roughly half by treatment with the EBS wild type.

These data suggest that synoviolin transcription is regulated by GABPa/β via the EBS.

### [Example 5] Identification of sites necessary for synoviolin expression in mouse embryos

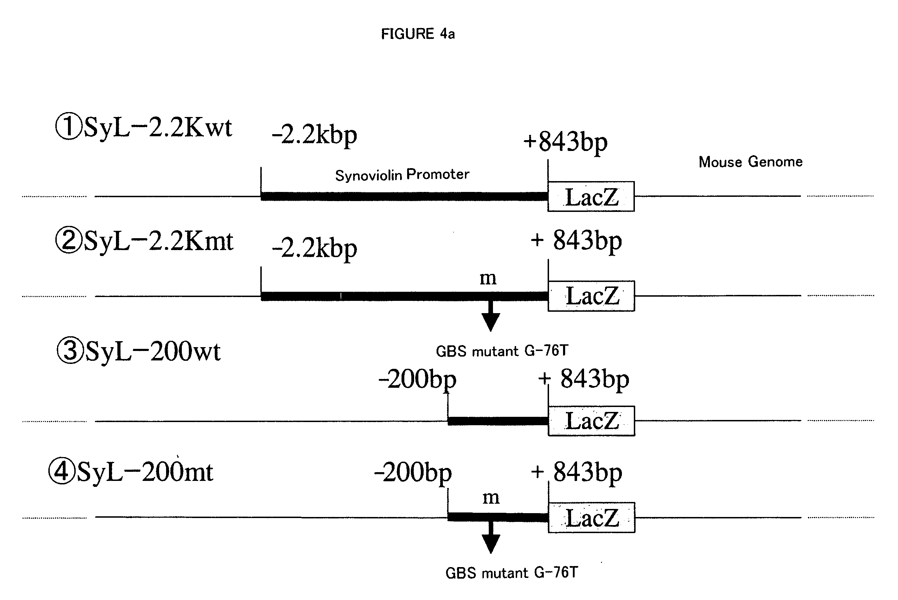

Next, a transgenic mouse in which a plasmid which the synoviolin promoter bound to LacZ was overexpressed was prepared to confirm the effects of EBS in vivo in mouse embryos. 4 Tgs were prepared having full-length 3k and 1k promoters and such promoters with single-nucleotide mutations, respectively (Figure 4a).

The transgenic (Tg) mouse constructions and Tg mice were prepared as follows.

Roughly 3k fragments were removed from SyG-2.2k-BV2 with NotI and NcoI, and inserted into SyTB/pbs to prepared SyL-2.2kwt/pbs. Similarly, SyL-2.2kmG-76T/pbs, SyL-200wt/pbs and SyL-200mG-76T/pbs were prepared using fragments extracted from SyG. The constructs were each purified using a QIAGEN Plasmid Kit (QIAGEN), and DNA linearized with ScaI was directly inserted by microporation into the nuclei of fertilized eggs of BDF mice (offspring from cross-breeding of C57BL/6N and DBA/2N), and transferred to the uterine tubes of surrogate mothers. Genomes were extracted from the tails of mice born from 8 to 9 uteri, which were confirmed from southern blotting to be Tg mice.

Embryos were X-gal stained to test synoviolin expression from these Tg mice. Expression of LacZ in knockout mouse embryos with LacZ knocked in was compared with expression in the 4 Tg mice prepared here.

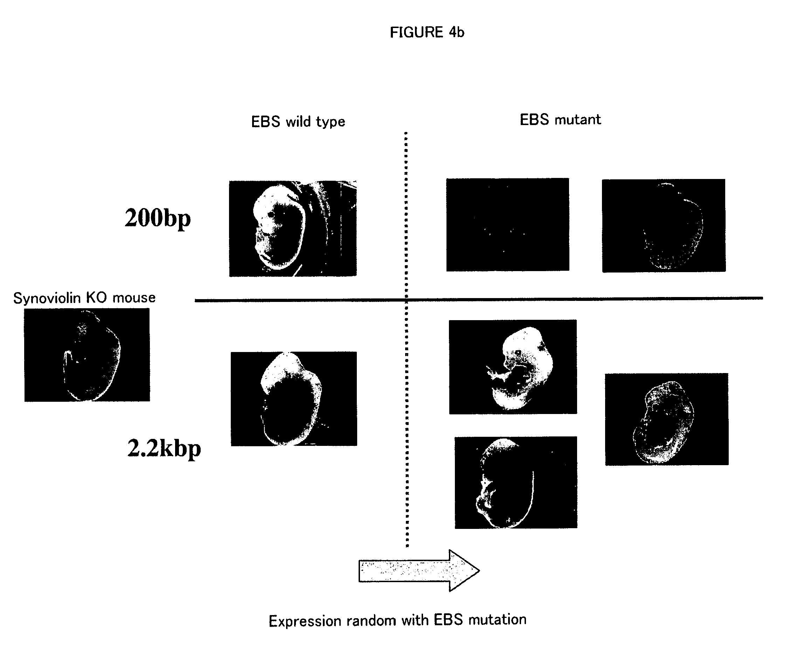

11.5 and 14.5 d.p.c. (days post coitus) synoviolin embryos were stained with LacZ. The transgenic mice having introduced 3k and 1k promoters exhibited staining in roughly the same locations as the hetero-knockout mice. Surprisingly, it was shown that in the transgenic mice having introduced 3k and 1k promoters with introduced single-nucleotide mutations, the expression sites were randomized (Figure 4b).

Similar results have been reported when a region necessary for transcriptional activation is eliminated (pax5, co111a2, etc.). This shows that EBS is a site necessary for transcription of synoviolin.

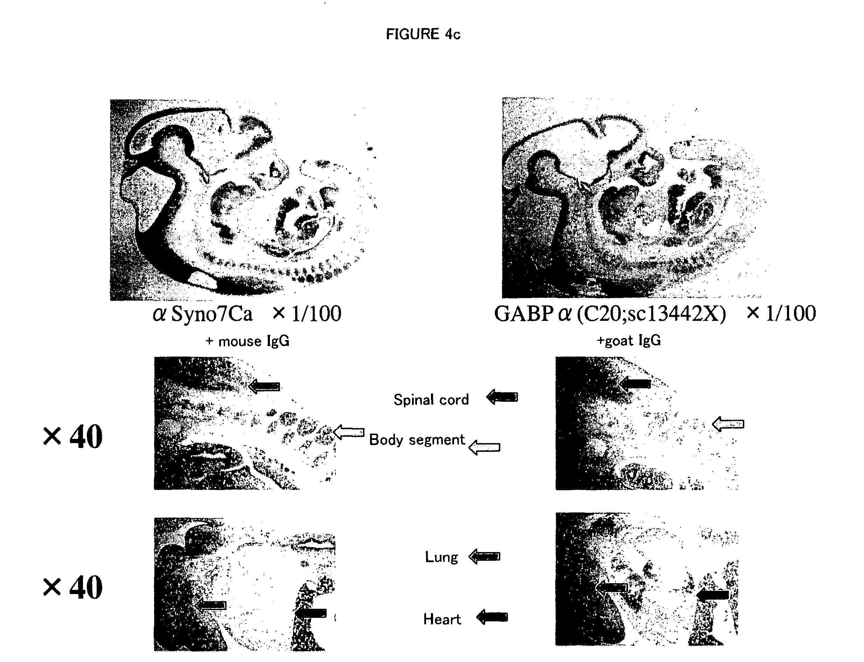

When this expression was compared to GABPa expression using a 13.5 d.p.c. embryo, expression was seen in roughly the same sites (Figure 4c). These results suggest that GABPa regulates synoviolin via the EBS in embryogenesis as well as in vitro.

### [Example 6] Expression of synoviolin in RA synovial cells

When the effects of GABPa via EBS in rheumatic synovial cells were studied, a supershift was found with GABPa as it was with the NIH3T3 extracts in a gel shift using nuclear extracts of synovial cells. Regulation of synoviolin expression was also investigated using an EBS decoy to test the significance of EBS in rheumatic synovial cells or in other words to confirm that transcriptional regulation by GABPa via EBS leads in turn to regulation of synoviolin expression and ultimately to regulation of synovial cell proliferation.

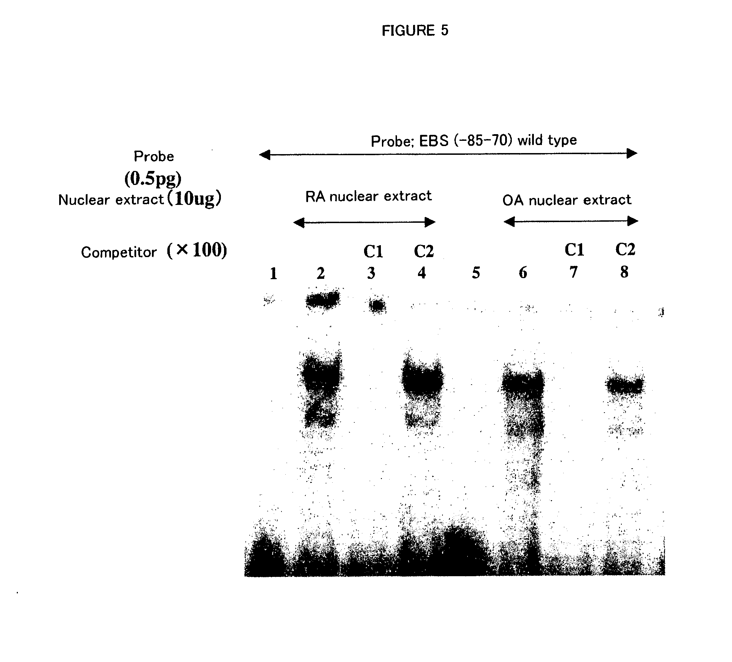

As a result, expression was reduced by roughly half (Figure 5).

These results show that GABPa/β is responsible for constitutive gene expression of synoviolin in RA synovial cells.

### [Example 7] Annexin V staining and FACS analysis

NIH3T3 cells were treated with trypsin, washed twice with chilled PBS, suspended in 1 x annexin-binding buffer (Vybrant apoptosis assay kit, Invitrogen), and prepared to a concentration of 1 x 10⁶ cells/ml. 100 µl of cell suspension was used per test. 5 µg of FITC-labeled Annexin V and 1 µl of PI working solution were added and incubated for 15 minutes at room temperature, 400 µl of 1 x annexin-binding buffer was added and gently stirred, and this was analyzed with FACSCalibur (Becton Dickinson).

Since synoviolin is resistant to apoptosis induced by ER stress, the following experiment was performed to clarify the relation between synoviolin levels and apoptosis.

NIH3T3 cells were prepared and after 24 hours siRNA (25 nM) of GFP or synoviolin was transfected into the NIH3T3 cells using Lipofectamine 2000 (Invitrogen), and incubated for 84 hours. An EBS decoy ODN was prepared as above and transfected into the NIH3T3 cells.

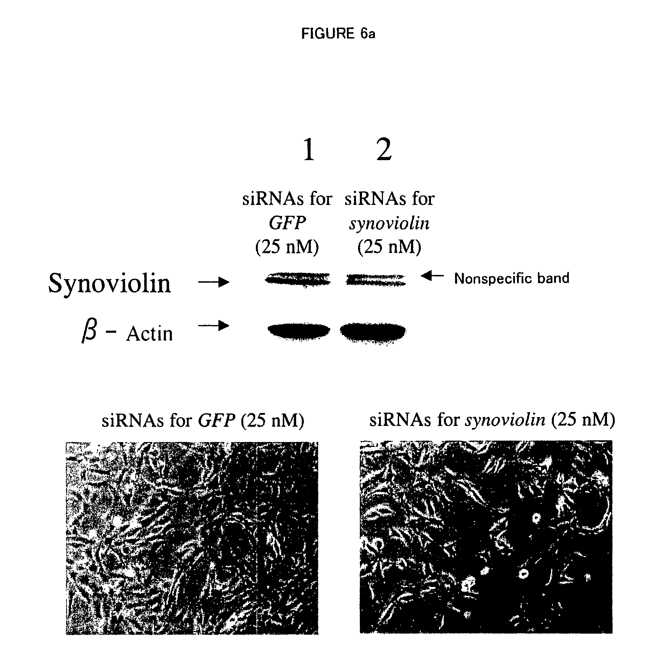

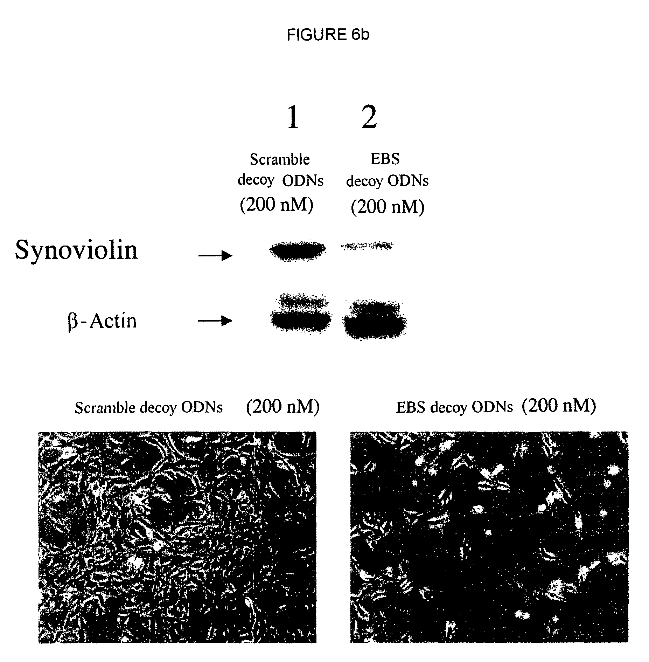

As a result, apoptosis was induced when the level of synoviolin expression was depressed in NIH3T3 cells using synoviolin RNAi (Figure 6a). In Figure 6a, the upper panel shows the results of western blotting, while the lower panel shows photomicrographs of NIH3T3 cells (100x). Similar, when the effect of transcriptional inhibition of synoviolin using EBS decoy nucleic acid was investigated, apoptosis was induced in NIH3T3 cells (Figure 6b). In Figure 6b, the upper panel shows the results of western blotting, while the lower panel shows photomicrographs of NIH3T3 cells (100x).

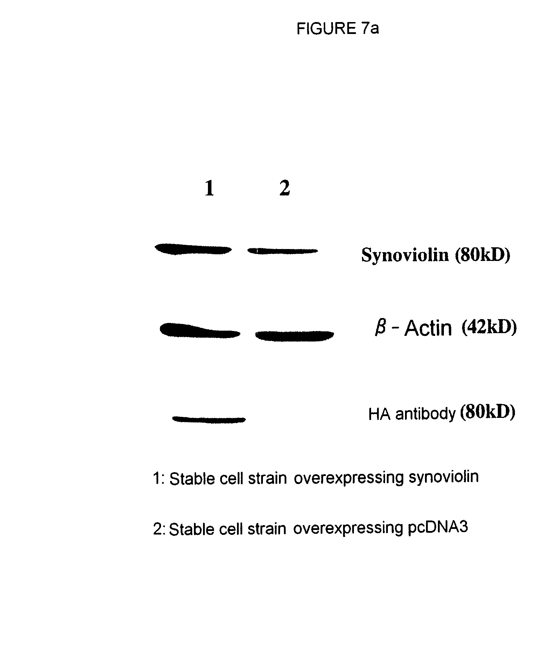

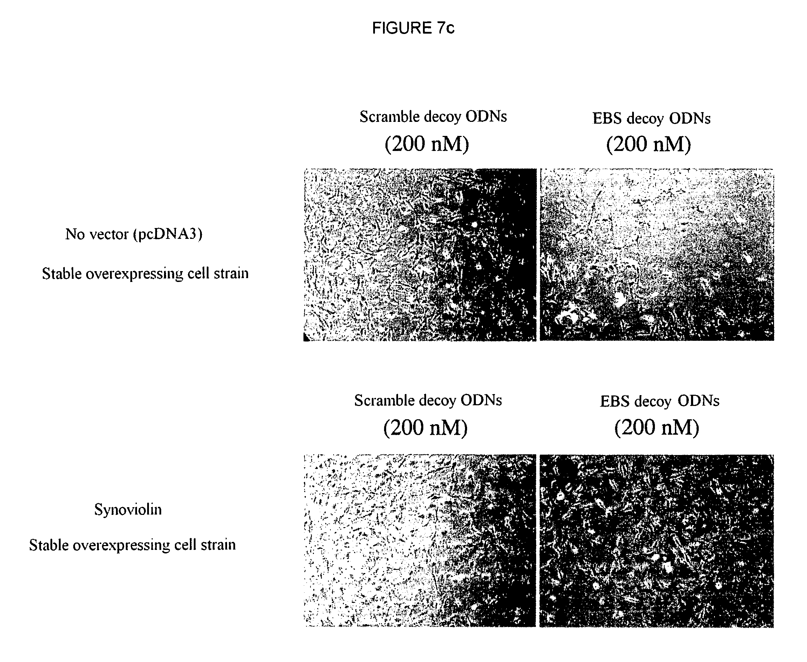

NIH3T3 cells overexpressing synoviolin were also prepared (Figure 7a) and used to investigate the effects of EBS decoy nucleic acid.

To establish a stable cell line overexpressing synoviolin, NIH3T3 cells were transfected with Lipofectamine 2000 (Invitrogen), and 24 hours later were diluted 10 times with fresh growth medium and subcultured. The next day, selection medium (containing 0.5 µg/ml G418) was added, and a clone stably expressing a HA-Synoviolin-HAHA/pcDNA3 expression vector was obtained. A HA-pcDNA3 empty vector was used as a control. To confirm expression from the plasmid in each cell line, western blotting was performed using antibodies to the HA tag.

Apoptosis induction with EBS decoy ODNs was tested as follows.

84 hours after transfection of EBS with FUGENE6 (Roche) reagent, the cells were collected, placed in a microtube and labeled with Annexin V-FITC. The distribution of live cells and cells which had undergone apoptosis was analyzed be FACS analysis. Western blotting was also performed using synoviolin antibodies. Beta-actin antibodies were used as a control, and HA antibodies were used to confirm stable expression.

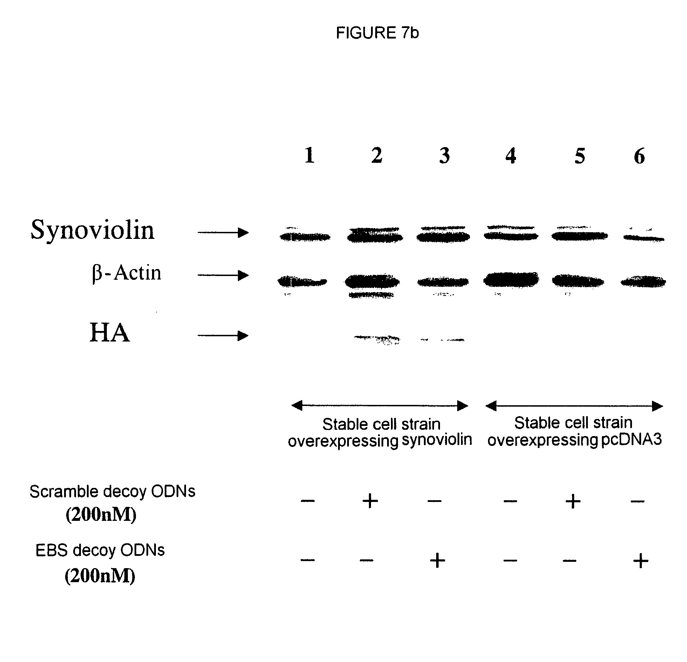

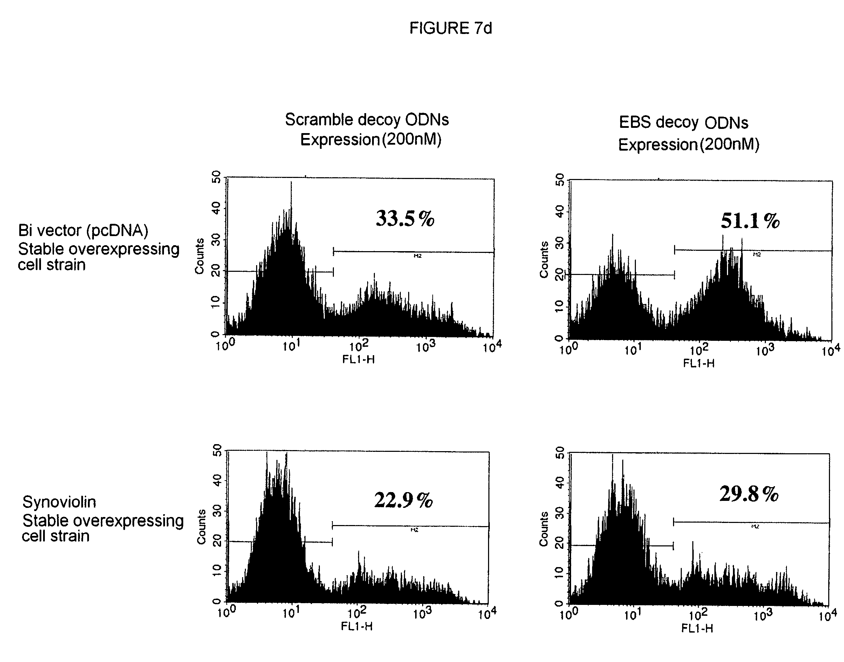

The results are shown in Figure 7. Cells overexpressing synoviolin were resistant to apoptosis caused by EBS decoy nucleic acid (Figure 7b, Figure 7c). Moreover, in an assay of apoptosis by FACS using Annexin V, the apoptosis rate of the normal NIH3T3 cells was 51.1% while that of the cells overexpressing synoviolin was only 29.8% (Figure 7d). Figure 7c is a photomicrograph of NIH3T3 cells at a magnification of 100.

These results show that when transcriptional regulation via EBS is suppressed, synoviolin expression is also suppressed and apoptosis is induced in NIH3T3 cells.

### INDUSTRIAL APPLICABILITY

A synoviolin promoter is provided by the present invention. The promoter of the present invention is useful because it regulates synoviolin expression, and also can be used for treating various disorders (such as rheumatism) by causing expression of useful genes at sites of synoviolin expression.

### Sequence Listing Free Text

SEQ ID: NO 3: Synthetic DNA

SEQ ID: NO 4: Synthetic DNA

SEQ ID: NO 5: Synthetic DNA

SEQ ID: NO 6: Synthetic DNA

SEQ ID: NO 7: Synthetic DNA

## Claims

1. A promoter of the synoviolin gene, comprising at least the nucleotide sequence of nucleotides 2120-2130 in the nucleotide sequence represented by SEQ ID: NO 1 or 2.

2. A promoter of the synoviolin gene, comprising any nucleotide sequence selected from a group consisting of at least the nucleotide sequences of the regions of nucleotides 1-2201, 969-2201, 1142-2201, 1699-2201, 1880-2201, 2002-2201, 2094-2201 and 2118-2201 in the nucleotide sequence represented by SEQ ID: NO 1 or 2.

3. A promoter of the synoviolin gene, comprising any nucleotide sequence selected from a group consisting of at least the nucleotide sequences of the regions of nucleotides No. 1-3043, 969-3043, 1142-3043, 1699-3043, 1880-3043, 2002-3043, 2094-3043 and 2118-3043 in the nucleotide sequence represented by SEQ ID: NO 1.

4. A promoter of the synoviolin gene, comprising any nucleotide sequence selected from a group consisting of at least the nucleotide sequences of the regions of nucleotides 1-3092, 969-3092, 1142-3092, 1699-3092, 1880-3092, 2002-3092, 2094-3092 and 2118-3092 in the nucleotide sequence represented by SEQ ID: NO 2.

5. The promoter of (a) or (b) below:
(a) A promoter of the synoviolin gene, comprising a nucleotide sequence having a deletion, substitution or insertion in some region of the nucleotide sequence of the promoter according to any one of Claims 1 to 4, and having promoter activity;
(b) A promoter of the synoviolin gene, comprising a nucleotide sequence which hybridizes under stringent conditions with a sequence complementary to the nucleotide sequence of the promoter according to any one of Claims 1 to 4, and having promoter activity.

6. A gene expression cassette comprising the promoter according to any one of Claims 1 to 5, a target gene for expression and a terminator.

7. A recombinant vector comprising the gene expression cassette according to Claim 6.

8. A transformant comprising the recombinant vector according to Claim 7.

9. A method for regulating transcription activity wherein the activity of the promoter according to any one of Claims 1 to 5 is inhibited or enhanced.

10. The method according to Claim 9, wherein inhibition or enhancement of the promoter activity inhibits or enhances the binding activity of a transcriptional factor.

11. The method according to Claim 10, wherein the binding site of the transcriptional factor is the Ets binding site.

12. The method according to Claim 10 or 11, wherein the transcriptional factor is any selected from the group consisting of GABPa, GABPβ, a complex of GABPa and GABPβ, Ets1, Pea3, Tel and Fli-1.
